# EUROPEAN PATENT APPLICATION

(11) **EP 1 344 458 A1**
(43) Date of publication of application: **17.09.2003**
(21) Application number: 02005607.3
(22) Date of filing: 12.03.2002
(51) Int. Cl.: A23K 1/00, A23L 1/03, A61K 35/74, C12N 1/04

(54) **Probiotic delivery system**

(71) Applicant: Société des Produits Nestlé S.A., 1800 Vevey (CH)
(72) Inventor: Ubbink, Johan Bernard, La Claie-aux-Moines, 1073 Savigny (CH); Schaer-Zammaretti, Prisca, 8051 Zuerich (CH); Cavadini, Christoph, 1801 Le Mont-Pelerin (CH)
(74) Representative: Wavre, Claude-Alain

(57) **Abstract**

The present invention relates to a probiotic delivery system that is preferably added to a food product. In particular, the invention shows that compacted pellets having a volume of at least 0.02cm³, that comprise, besides viable micro-organisms, arbitrary or eligible components, such as fillers, binder, plasticizer, other functional ingredients and a coating may be added to semi-moist, moist or semi-dry products. The micro-organisms remain viable for a longer time than commercially obtainable preparations of probiotics.

## Description

### Probiotic Delivery System

The present invention relates to pellets comprising viable micro-organisms and a coating, to a delivery system of probiotics, to the use of the pellets as a delivery system of probiotics in a food product and to a process for obtaining a delivery system to supplement food products with. The invention further relates to a food product comprising the pellets.

### The Background Art

Probiotic micro-organisms (hereinafter: probiotics) are living micro-organisms, which upon ingestion in certain numbers, exert health benefits beyond basic nutrition. The beneficial effects that probiotics may induce are numerous and form part of the knowledge of the skilled person. As few examples one may mention the reduction of lactose intolerance, the inhibition of pathogenic bacteria and parasites, the reduction of diarrhoea, activity against *Helicobacter pylori,* the prevention of colon cancer, the improvement or prevention of constipation, the *in situ* production of vitamins, the modulation of blood lipids, and the modulation of host immune functions.

Above described beneficial effects are generally valid for very specific strains of micro-organisms colonising the intestines of mammals, for examples companion animals, such as pets, and also for humans.

Therefore, there is considerable interest in including probiotics into foodstuffs. For example, many fermented milk products, such as yoghurts, which contain probiotics are commercially available.

Similarly, for animals, there has been interest in including probiotics into animal feed. This holds true at least for feed aimed at livestock as well as for pet food.

Many probiotics exhibit their beneficial effect mainly when they are alive. Hence, if they are added to a food product, probiotics are meant to survive the shelf life of the food, and even more, upon consumption of the food, the passage through the gastro-intestinal tract as far as the place of colonization.

Therefore, the state of the art is concerned with the problem of supplying probiotics together with foodstuff and/or pet food and providing a prolonged lifetime of the added probiotics. In particular, probiotics are very sensitive to temperature and moisture as in a moist or semi-dry food, while they are comparatively stable in a dry environment, for example, characterized by a water activity (Aw) below 0.2. The prior art is, therefore, concerned with the conservation of living biomass in a humid, moist or even liquid environment at ambient and higher temperatures.

EP 0 862 863 provides a ready-to-eat cereal product comprising a gelatinized starch matrix including a coating or a filling which comprises a probiotic. Accordingly, spray-dried probiotics are mixed into a carrier substrate, which may be water, fat or a protein digest, and the mixture is then essentially sprayed onto the cereal product. Of course, the cereal product must itself have low water activity to ensure a prolonged lifetime of the probiotics.

EP 0 704 16 is concerned more concretely with the preservation of lactic acid bacteria in moisture food. The spray-dried bacteria are added to a composition comprising fats, fermented milk powder and saccharides. This composition is then indended as the filling of a confectioney product. This invention avoids the detrimental effects of water by embedding the probiotics in a matrix rich in fat or oil and therefore risks to shift a balanced nutritional composition of a food product to the negative.

EP 0 180 743 discloses micro-organisms that are suspended in an oil phase and are encased by at least one protective layer, which is water-soluble (water-soluble derivatives of cellulose or starch, gums or pectins).

A good stability of micro-organisms in a micro-encapsulated form is commercially available from Cerbios-Pharma SA, Bioferment, Lugano, Switzerland under the product name of LBC ME 10, for example . A microscopic view on a cut of the small capsules (diameter about 700µm) shows that probiotics attached to a carrier are coated by several thin layers that protect the probiotics. These products are characterised by a relatively high stability also in moisture environments, but are also expensive to produce, since several layers must be added, to avoid water entering the micro-capsules.

Therefore, a need exists for a delivery system of probiotics, which provides, with respect to the existing prior art, a still prolonged life span of the probiotics in a liquid, moist or semi-moist environment.

It is, in particular, the challenge of the present invention to provide stable probiotics or a probiotic delivery system, added to a food product that has itself an Aw value above the optimal value for probiotics to survive. Furthermore, the probiotics should preferably be provided in a form, which does not substantially differ from or deteriorate (from a nutritional and organoleptic point of view) the food product to which they are added.

### Summary of the Invention

Remarkably, it was found that by compacting dried micro-organisms together with a matrix, which may consist of dried food material, and by coating the pellets with a food-grade moisture barrier an excellent stability over storage time is obtained.

Consequently, in a first aspect, the present invention provides a pellet comprising a compacted inner matrix and at least one coating, wherein the inner matrix comprises viable micro-organisms and the coating comprises a moisture barrier, characterised in that the pellet has a volume of at least 0.02cm³.

In a second aspect, the present invention provides pellets within a food product, wherein the pellets are the pellets according to the invention and the inner matrix of the pellets comprises at least one component of the food product.

In a third aspect, the present invention provides a delivery system of probiotics, which comprises the pellets according to the invention.

In a fourth aspect, the present invention provides the use of the pellets according to the invention as a delivery system for probiotics in a moist, semi-moist, or semi-dry food product.

In a fifth aspect, the present invention provides a process for obtaining a pellet to supplement a food product with viable micro-organisms, which comprises the steps of mixing a preparation of micro-organisms and further components, drying the mixture to an Aw below 0.3, compacting the mixture under pressure to obtain pellets comprising a volume of at least 0.02cm³, and coating the pellets with a moisture barrier.

In a sixth aspect, the present invention provides a food product comprising the pellets according to the invention, wherein the food product and the inner matrix of the pellet share at least one component, ingredient or constituent.

In a last aspect, the present invention provides a particulate food, food additive, supplement or pharmaceutical product consisting of a mixture of at least two types of particles, one being the probiotic pellet according to the invention.

An advantage of the present invention is that it provides a significant improvement of the stability of probiotic micro-organisms applied in semi-dry and/or humid particulate foodstuffs.

Another advantage of the present invention is that the processing is easy and straightforward.

Yet another advantage of the present invention is that it provides a suitable delivery vehicle for further functional ingredients, in particular also prebiotic fibres, which in turn may improve the physico-chemical characteristics of the pellet according to the invention.

In the figures,

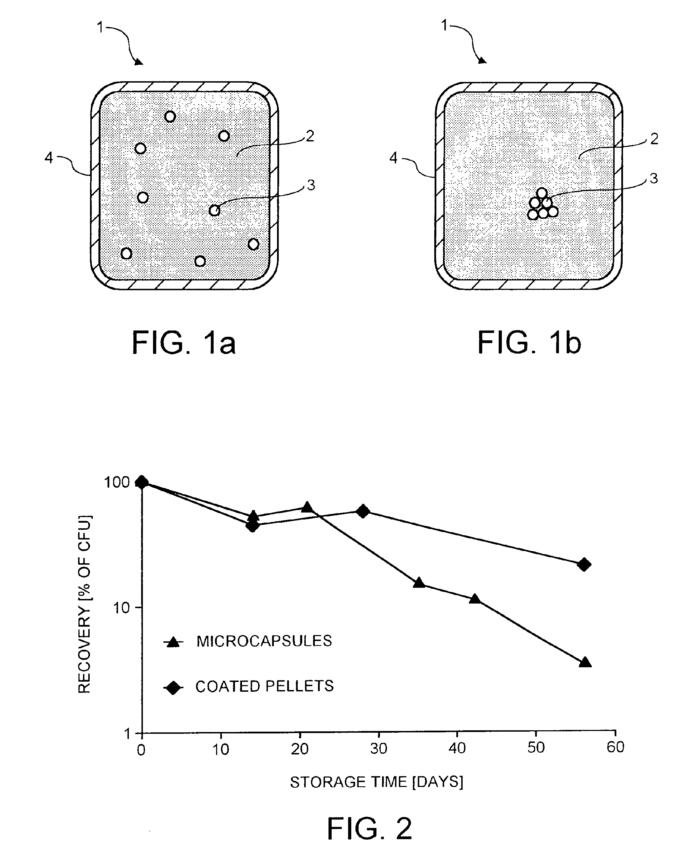

Figure 1 schematically illustrates an example of the pellet (1) according to the invention. The pellet (1) comprises an inner matrix (2), which comprises probiotics (3). The pellet further comprises a moisture barrier (4). The Figures 1a and 1b distinguish different embodiments, wherein the probiotics are homogenously dispersed (Figure 1a) or accumulated in the centre (Figure 1b). It should be noted that the shape and the ratios between thickness of coating, inner matrix and probiotic preparation is arbitrary and only serves the purpose of illustration.

Figure 2 shows the storage stability (recovery in % of cfu/g versus storage time) of *Enterococcus faecium* stored at 30°C and 70% rH (relative humidity). The figure distiguishes (•) values obtained with the commercially available micro-capsules obtained from Cerbios-Pharma, Lugano, Switzerland under the designation SF68 and the coated pellets according to the invention (◆).

### Detailed Description of the Invention

Within the context of this specification the word "comprises" is taken to mean "comprises, among other things". It is not intended to be construed as "consists only of".

Within the context of the present invention, the term "food product" is intended to encompass any consumable matter. Hence, it may be a product intended for the consumption by humans, but the term also encompasses products to be consumed by animals, for example pets, such as dogs, cats, rabbits, guinea pigs, mice, rats, birds (for example parrots), reptiles and fish (for example goldfish). However, the term also includes food to be consumed other domesticated animals, for example feed products for livestock, for example, cattle, horses, pigs, sheep, goats, buffaloes, camels, and the like.

The term "pellet", in the context of the present invention, is not intended to refer to any specific form. In the contrary, a "pellet", in the sense of the present invention, may assume any form obtainable by compaction. For example, a pellet may have the form of a sphere, cube, pyramid, tablet or any classic, modified or complex three-dimensional form. Furthermore, fancier forms may be conceived. For example, if the pellets are intended as a probiotic delivery system for pet-food, they may have the form of bones, rods, rings, the form of animals, for example mice, or other objects. The compaction techniques of today allow the preparation of almost any three-dimensional structure.

In the context of the present invention, the term "probiotic" is intended to refer to any micro-organism that is wished to be consumed owing to any beneficial effect it may have on its consumer.

In the context of the present invention, the term "moisture barrier" refers to any substance that may be used to coat the pellets as defined above and is useful to slow down the water absorption by the pellets to be coated.

In the context of the present invention the following Aw values for defining "moisture" are used as practical approach: moist denotes an Aw of 0.7 or above, semi-moist an Aw between 0.5 and 0.7 and semi-dry and Aw between 0.3 and 0.5.

In the context of the present invention, the word "matrix" in the expression "inner matrix" is not intended to be restricted to any specific component or selection of components. Of course, it usually comprises food-grade ingredients, the consumption of which is nutritionally safe. However, one of the advantages of the present invention is the very flexibility the skilled person is given with respect to the matrix. The matrix may serve as a carrier for the probiotics, optionally together with a binder.

The word "binder", in the context of the present invention, may refer to any food-grade substance that has the property of giving other food ingredients the ability to be compacted. It is generally understood that binders have the ability to be plastically deformed during compaction, thereby delivering a sufficiently durable tablet structure. In addition, binders may have adhesive, gluey or sticky properties.

The word "plasticizer", in the context of the present invention, may refer to any foodgrade substance that has the property of softening one or more of the other ingredients used in preparing the compacted pellet. Preferably, plasticizers are used that have the capacity of bedewing or wetting the surface of other components of the inner matrix and by this way support the compaction of the inner matrix even at a low water activity.

The word "functional food", in the context of the present invention, refers to any food product, or any food ingredient that provides to the consumer any benefit that goes beyond basic nutrition. Probiotics (see above) that may have beneficial effects on the composition and metabolic activity of the gut microlfora of the host and on its immune system, may serve as an example. Likewise, prebiotics, fiber, vitamins, anti-oxidants, psycho-stimulating molecules, such as caffeine, may serve as a purely arbitrary, exemplary but illustrative choice of possible "functional food" ingredients.

The term "further components", in the context of the further components of the inner matrix, refers to all other components than bacteria. In case that the bacteria are attached to a specific carrier, this carrier material is likewise not part of the "further components".

All lists of ingredients or parts of the pellet or probiotic delivery system given within this description are regarded as non-exhaustive lists. It will always be possible, though not mandatory, to select any combination of the ingredients or substances of one or all of these lists.

Percentages are given in percent by weight, unless otherwise indicated.
Preferably, in an embodiment the pellet according to the present invention has a volume of 0.01 to 100cm³, preferably 0.02 to 50cm³, more preferably 0.125 to 30 cm³ and most preferably 0.3 to 8cm³. For example, the pellet has a volume of 0.6 to 2.25 cm³.

In another embodiment according to the present invention, the inner matrix of the pellet according to the present invention before or shortly after the coating, is characterised by a water activity of below 0.3. Preferably, the water activity is below 0.2, more preferably about, equal to or below 0.1. For example, the water activity is in the range of 0.01 to 0.09.

In a further embodiment, the inner matrix of the pellet according to the present invention has an envelope density of more than 0.8g/cm³. Preferably, it has an envelope density of above 1.1 g/cm³, more preferably above 1.3 g/cm³.

The envelope density is a measurement that indicates the specific weight of an object including pore spaces up to the plane of the surface. This quantitiy is specifically suitable for tablets or compacted entities, and may be assessed with a Micromeritics® Geo Pyc 1360 apparatus, for example.

Another way of expressing specific weight of compacted entities, for example is the absolute density. The absolute density of the uncoated pellet is preferably above 1g/cm³, more preferably above 1.2 g/cm³ and most preferably above 1.5 g/cm³. The absolute density may be assessed with a Micromeritics® Accu Pyc 1330 apparatus, for example.

Definitions of envelope and absolute densities are known to the skilled person but may be derived, for example from Webb, PA; Orr, C. Analytical methods in fine particle technology. Micromeritics Instrument Corporation, Norcross, GA, 1997.

Generally, it may be said that the envelope density is the overall density of a body as determined from its volume including closed and open pores up to the plane of the surface. The absolute density is the density of a body as determined from its volume inlcuding closed pores (but excluding the open pores). The matrix or real density (not indicated in the embodiments above) is the density of a body as determined by the volume of the matrix excluding both open and closed pores.

Generally, the envelope density of the uncoated pellets is between about 50% and 100%, preferably between 70% and 100% and more preferably between 80% and 100% of the absolute density of the uncoated and uncompacted components of the inner matrix.

In still another embodiment, the pellet according to the present invention comprises 10⁵ to 10¹² viable micro-organisms (cfu). Preferably, it comprises 10⁵ to 10¹¹ cfu, more preferably 10⁶ to 10¹⁰ cfu. For example, the pellet according to the invention may comprise 10⁶ to 10⁸ cfu.

The above values are dependant on the size of the pellet and the number of cfu added. The recommended values of cfu may also be expressed in g of pellet, disregarding the size of the pellet. Hence, the pellet according to the invention preferably comprises 10⁵ to 10⁹, more preferably 10⁶ to 10⁸ cfu/g.

In stil a further embodiment, the inner matrix of the pellet according to the present invention further comprises ingredients selected from the group of digestible starches, resistant starches, other fibre, milled cereals, dried and milled vegetables, cellulose and cellulose derivatives, pet food, maltodextrin, chicory flour, protein isolates, yeast extracts and mixtures thereof.

In yet a further embodiment, the coating of the pellet according to the invention comprises a food-grade moisture barrier.

In an embodiment of the process according to the invention, the further components comprise at least part of the ingredients of the food product.

Without wishing to be bound by theory it is postulated that by adding dried probiotics to food material or specific food grade ingredients, drying, compacting them to relatively large particles or pellets (≥ 0.02cm³), and coating them with a material that serves as a moisture barrier, a high stability of the encapsulated probiotics is reached. This may be so in part because the ratio of volume and surface is much better exploited than in encapsulated or dried probiotics so far known. The compaction and the moisture barrier further support the optimised ratio and allows a storage stability of probiotics in a moisture environment that was so far not achieved.

In order to prepare the pellets according to the present invention, a single or a mixture of different possible micro-organisms, the further components of the inner matrix and the hydrophobic substance may be selected.

As a micro-organism, any micro-organism may be selected. Preferably, a micro-organism exerting beneficial effects on health and welfare on humans or animals, such as pets, for example cats or dogs, lifestock animals, for example, pigs, cattle, buffaloes, sheep or goats is selected. Preferably, the micro-organism is a probiotic micro-organism.

The literature mentions some of the micro-organisms that may be used to carry out the present invention, for example in EP 0 862 863A2, in particular on page 3.

Examples of suitable probiotic micro-organisms include yeasts such as *Saccharomyces, Debaromyces, Candida, Pichia* and *Torulopsis*, moulds such as *Aspergillus, Rhizopus, Mucor,* and *Penicillium* and *Torulopsis* and *bacteria* such as the genera *Bifidobacterium, Bacteroides, Clostridium, Fusobacterium, Melissococcus, Propionibacterium, Streptococcus, Enterococcus, Lactococcus, Kocuria, Staphylococcus, Peptostrepococcus, Bacillus, Pediococcus, Micrococcus, Leuconostoc, Weissella, Aerococcus, Oenococcus* and *Lactobacillus*. Specific examples of suitable probiotic micro-organisms are:
Aspergillus niger, A. oryzae. Bacillus coagulans, B. lentus, B. licheniformis, B. mesentericus, B. pumilus, B. subtilis, B. natto, Bacteroides amylophilus, Bac. *capillosus, Bac. ruminocola, Bac. suis, Bifidobacterium adolescentis, B. animalis, B. breve, B. bifidum, B. infantis, B. lactis, B. longum, B. pseudolongum, B. thermophilum, Candida pintolepesii, Clostridium butyricum, Enterococcus cremoris, E. diacetylactis, E. faecium, E. intermedius, E. lactis, E. muntdi, E. thermophilus, Escherichia coli, Kluyveromyces fragilis, Lactobacillus acidophilus, L. alimentarius, L. amylovorus, L. crispatus, L. brevis, L. casei, L. curvatus, L. cellobiosus, L. delbrueckii ss. bulgaricus, L. farciminis, L. fermentum, L. gasseri, L. helveticus, L. lactis, L. plantarum, L. johnsonii, L. reuteri, L. rhamnosus, L. sakei, L. salivarius, Leuconostoc mesenteroides, P. cereviseae (damnosus), Pediococcus acidilactici, P. pentosaceus, Propionibacterium freudenreichii, Prop. shermanii, Saccharomyces cereviseae, Staphylococcus carnosus, Staph. xylosus, Streptococcus infantarius, Strep. salivarius ss. thermophilus, Strep. thermophilus, Strep. lactis.*

For example, a probiotic strain or strains may be selected from the group comprising *Bacillus licheniformis* (DSM 5749), *B. subtilis* (DSM 5750), *Bifidobacterium lactis* (DSM20215), strains of *Enterococcus faecium* (e.g. NCIMB 10415; NCIMB 11181; NCIMB 30098; DSM 3520; DSM 4788; DSM 4789; DSM 5464; DSM 7134; CECT 4515), *E. mundtii* (CNCM MA 27/4E), strains of *Saccharomyces cereviseae* (e.g. BCCM / MUCL 39885; CBS 493 94; CNCM I-1077; CNCM I-1079; NCYC Sc47), *Lactobacillus casei* (NCIMB 30096), *L. farciminis* (CNCM MA 67/4 R), *L. johnsonii* (I-1225 CNCM), *Lactobacillus paracasei* (I-2116 CNCM), *L. plantarum* (CNCM I-840), *L. rhamnosus* (DSM 7133), *P. acidilactici* (CNCM MA 18/5 M), *Streptococcus infantarius* (CNCM I-841), *Streptococcus thermophilus* (TH4, Chr. Hansen, DK), and mixtures thereof, for example.

Further examples of probiotic species with exemplary, deposited strains of the species according to the present invention may be selected from the group comprising *Lactobacillus reuteri* (CNCM I-2452, CNCM I-2448, CNCM I-2450, CNCM I-2451), *Lactobacillus rhamnosus* (CNCM I-2449), *Lactobacillus acidophilus* (CNCM I-2453), and mixtures thereof. The strains mentioned in this paragraph may be particularly suitable for pets.

The micro-organisms are preferably in a dried form, or for example in a spore form for micro-organisms which form spores. The drying of micro-organisms after production by fermentation is known to the skilled person. For example, EP 0 818 529 (SOCIETE DES PRODUITS NESTLE), where a drying process of pulverisation is described, or WO 0144440 (INRA). Usually, bacterial micro-organisms are concentrated from a medium and dried by spray drying, fluidised bed drying, lyophilisation (freeze drying) or another adequate drying process. For example, micro-organisms are mixed with a carrier material such as a carbohydrate, for example sucrose, lactose or maltodextrin, a lipid or a protein, for example milk powder during or before the drying. If a carrier material is used, it may also form part of the inner matrix.

However, the micro-organisms need not necessarily be present in a dried form. It may also be conceived to mix them directly after fermentation with the further components of the inner matrix (see below) and to perform a drying process thereafter. Such an approach could be deducted from WO ....... (SOCIETE DES PRODUITS NESTLE).

Furthermore, the further components of the inner matrix may be selected. One of the advantages of the present invention is the high flexibility and variety with respect to the further components of the inner matrix. This high variety is reflected by the lists of possible molecules or functions that may be added to the inner matrix.

Therefore, the further components may be regarded as "fill-up" or "filler" components and their choice is almost arbitrary and freely eligible. In case that the micro-organism is already mixed with further ingredients, for example carrier materials or protective agents, it is even possible that no further component must be selected.

Generally, if further components of the inner matrix are selected, substances with a high hygrocapacity are generally advantageous with respect to the ability of the coated pellet to retard the increase of the internal water activity. Even if water is absorbed by the inner matrix of the pellet, the water activity remains relatively low because of the high capacity of the inner matrix to absorb moisture. Polymeric carbohydrates, for example, have a high hygrocapacity.

Preferably, the further components of the inner matrix may be selected in a way that compaction of the inner matrix is possible. Generally, this may be also achieved by a suitable binder and/or plasticizer.

For the sake of convenience and to avoid confusion in view of the high variety or choice in the selection of the further components of the inner matrix, these are exemplary discussed as fillers, functional ingredients, lubricants, plasticizers and binders. It is understood that these classes do not cover completely different ingredients, but there may be an even substantial overlap. For example, some plasticizers may also serve as binders, or fillers may also comprise functional ingredients, such as fibres, for example.

Fillers, that may help to increase the volume may be selected from the group comprising starches; resistant starches, low molecular weight sugars, for example, lactose, dextrose, sucrose, and/or mannitol, microcrystalline cellulose, modified starches, for example, amylodextrin hydrogen octenylbutanedioate; and/or starch n-octenylsuccinate, proteins, for example milk-, pea-, soy-, meat-, poultry-, gluten-protein, hydrocolloids, milled cereals, dried and milled vegetables, animal meal, milk powder, cocoa powder, milled biscuit, or mixtures thereof.

Functional ingredients may be selected to provide further benefit to the pellet or delivery system according to the present invention. They may comprise, apart from the probiotics, prebiotic fibres, for example fructo-oligosaccharides (FOS), polyfructoses, for example, inulin or levan, resistant starches, for example retrograded starch, dextrans, arabinogalactans, for example acacia gum, galactomannans, for example guar, galactooligosaccharides, isomaltooligosaccharide, maltooligosaccharide, maltodextrins, and mixtures thereof, for example.

Fibres may be differentiated in soluble and non-soluble fibre. Examples of soluble fibers comprise inulin, pectin, 6-glucans (small cellulose-type branched glucose polymers), gumarabic, tragacanth, mucilages, guar and locust bean gum, agar, carageenans, alginates, xanthan and the like. Most of these soluble fibres are fermentable for the largest part. Examples of insoluble fiber comprises cellulose (for example derived from oat hull, soy-beans, cereal bran) and hemicellulose (mostly branched arabino-xylans or galactans, e.g. from cereals, potatoes or soybeans). Most of these insoluble fibres are partly fermentable or non-fermentable.

Functional ingredients may also comprise trace elements, minerals, vitamins, antioxidants, sterols, antioxidants, fatty acids, proteins, for example enzymes, and/or other functional molecules.

Examples of vitamins and/or antioxidants may be selected from the group comprising carotenoids, such as lycopene, α-, β-, or γ-carotin, xanthophylls, vitamin A1, vitamin A2, tocopherols, for example vitamine E , vitamine C, and mixtures thereof.

Examples of fatty acids may be selected from the group comprising long-, medium chain saturated or unsaturated, mono-, di-, or triacylglycerols, and mixtures thereof, for example.

Examples of enzymes may be selected from the group comprising proteases, peptidases, lipases, hydrolases, and cocktails thereof, for example.

Other functional molecules may be selected from the group of bacteriocins, chondroitin sulfate, soy isoflavones, nucleotides, nucleosides, isothiocyanates, cruciferous extracts, for example from broccoli, sulfloraphane, and mixtures thereof, for example.

Ingredients or molecules that have other functions may be added to the inner matrix. These ingredients or molecules may enhance intestinal functions, maintain or enhance skin integrity, prevent skin damage (for example, UV induced) and stress response, improve coat conditions, prevent infection, maintain or improve oral health, enhance and/or maintain vision, prevent allergy, modulate immune functions, prevent obesity, provide weight control, lower the risk of diabetes and/or artherosklerosis, control triglycerides in blood and tissues, enhance nutrient absorption, improve brain development and enhance and/or maintain cognitive functions, prevent vascular disease, for example heart attack or stroke.

In addition or alternatively, ingredients or molecules may be provided that maintain and improve of kidney, liver and pancreas functionality, improve joint health, prevent arthritis, improve bone development during growth, improve or enhance maintenance of bone mass during adulthood, increase mineral (for example calcium) and vitamin absorption and utilisation from food, prevent and/or slow down osteoporosis, improve muscle growth, performance and/or recovery, have anti-inflammatory properties, improve breath, enhance immune functions, have anti-pathogenic activity and/or inhibitory activity.

Binders and/or plasticizers may be added to the components of the inner matrix, if necessary, to improve the compaction properties of the components of the inner matrix. It may be, however, that the further components and/or the micro-organisms are themselves sufficiently "sticky" or adhesive to allow for compaction. In this case, a specific "binding component" may be omitted. However, if one or several binders and/or plasticizers are added to support the formation of pellets by compaction, preferably food-grade ingredients are used.

Examples for plasticizers may be selected from the group comprising polyols (for example, glycerol, sorbitol, propyleneglycol), alcohols (for example, ethanol, propanol, butanol, isopropanol, isobutanol, butanediol).

Examples of binders may be selected from the group comprising suitable polysaccharides, for example starches (native starch, waxy maize starch, hydrolyzed starches, maltodextrins, pregelatinized starches), polyfructoses (chicory flour, inulin), hydrocolloids, polyvinylpyrrolidone, hydroxypropyl methylcellulose, for example.

Lubricants may serve the purpose of facilitating the release of the pellet from the tabletting, pelletting or briquetting mould by reducing the friction forces between the pellet and the forming mould and, if relevant, the tabletting punch. Moreover, they may promote the flow of the pellet premix in the hopper and in the tabletting or pelletting mould. Examples of lubricants may be selected from the group comprising stearic acid, stearic acid salts, stearic acid derivatives, talc, polyethylene glycols, surfactants, and waxes.

Since it is the objective of the present invention to add the pellets to a food product, it is an outstanding advantage that the inner matrix may comprise one, several or all components of the food product to which the pellets will be added.

For example, if the pellets are to be added to a pet food, the inner matrix may be supplemented, partially or totally, by the pet food to which the pellets will be added. Hence, pet food may just be milled and/or dried in either a cooked or uncooked state and thus used as part (for example filler) of the inner matrix of the pellets.

Accordingly, if the pellets are added to breakfast-cereals, the inner matrix may comprise cereals. Or, if the pellets are added to a snack as chips, for example, the inner matrix may comprise potato starch or other ingredients, such as flavours, used to prepare the chips. This inventive concept may be extended to any food product.

Also the moisture barrier coating the compacted components of the inner matrix may be selected. In principle, any food-grade substance having water repelling or impermeable properties may be selected. The skilled person is usually available to select one or mixtures of suitable moisture barriers. Nevertheless, a list is given below for illustration, from which at least one or mixtures may be selected.

Hence, suitable moisture barriers may be, for example, waxes (paraffin wax, beeswax (white and yellow), carnauba wax, candellila wax, microcrystalline wax, rice bran wax, cetyl ester wax, shellac, emulsifying wax, lanolin, hydrogenated castor oil, jojoba oil), fatty acids (for example, oleic acid, stearic acid, palmitic acid, lauric acid) and their salts (for example, sodium, calcium, magnesium, aluminium); fatty acid derivatives (for example, cetyl palmitate, acetic, lactic, citric and tartric mono and di glyceride fatty acids, sodium lauryl sulfate), esters of fatty acids (for example, isopropyl palmitate, isopropylmyristate), monoglycerides, diglycerides and triglycerides (for example, MCT oil, triglycerides based on coconut/palm kernel oil), derivatives of monoglycerides, diglycerides and triglycerides (for example, polyglyceric esters of fatty acids, propyleneglycol esters of fatty acids, vegetable oils and fats (for example, rapeseed, sesame, cornseed, nut, cottonseed, peanut, sunflower, linseed, olive, soy bean, cocoa butter) hydrogenated or hardened vegetable oils and fats, fractionated vegetable oils and fats, oils and fats of animal origin (for example, beef, poultry, pork, lamb; for example, beef tallow, lard), hydrogenated or hardened oils and fats of animal origin fractionated oils and fats of animal origin, dairy fats (for example, milkfat, fractionated milkfat, butterfat), proteins (for example, gluten, zein, sodium and calcium caseinate), phospholipids (for example, lecithin), carbohydrates (for example, cellulose and cellulose derivatives (for example, hydroxypropyl methylcellulose, ethylcellulose, methylcellulose, carboxymethyl cellulose), hydroxypropylated starch, carrageenans), sorbitan esters (for example, mono-oleate, -palmitate, -stearate, trioleate), mineral oils and fats (for example, paraffin), chocolate polyvinylalcohol, poly(3-hydroxy butyrate-co-3-hydroxyvalerate), poly(lactic acid), pharmaceutical glaze, latex, methacrylic acid copolymer, poloxamer, polyoxylethylene derivates, tocopherols, sterols, carotenoids, dimethicone, sucrose esters of fatty acids and sucroglycerides.

Mixtures and laminates of mentioned ingredients may comprise, for example, casein - acetylated monoglyceride, stearic acid, beeswax; casein, gelatin, soy protein, zein - fatty acid amylose ester; zein, albumen, casein, gelatin, soy protein - vegetable oil; nitrocellulose - wax; zein/vegetable vax-oil laminates.

The preparation of the pellets after selection of the micro-organism and the further components of the inner matrix may occur in any suitable way. A recent review of the state of the art in the technology and materials science of tablet compression is given in "Pharmaceutical Powder Compaction Technology", Alderborn G and Nyström C, eds, Marcel Dekker, New York (1996).

A few principle steps of preparation of the pellets may usually comprise the steps "mixing, drying, compacting and coating". The sequence of these steps may be varied in a way that corresponds to common sense. For example, the above-mentioned sequence may be modified to "mixing, compacting, drying and coating".

As a variation, ingredients of the inner matrix, comprising moist fillers, optionally binders or functional ingredients, besides probiotics, may be mixed and compacted, whereby a relatively high water content supports the compaction. Thereafter, the compacted pellet may be dried and coated.

Most of these steps, for example "mixing" and "drying", may be subdivided, for example "mixing only few of the ingredients, drying them, adding other ingredients to the mixture, compacting, drying again and coating".

As the above paragraphs illustrate, the preparation of the pellets according to the present invention is very flexible. It is preferred, however, that shortly before or at the moment of coating the compacted inner matrix, the inner matrix has a relatively low water activity (Aw), for example an Aw below 0.3, preferably below 0.2, more preferably below 0.15.

For example, a dry mix of the micro-organisms and the further components, is prepared by mixing all components. Then the mix may be dried to a Aw below 0.3, preferably below 0.2, more preferably below 0.15 and most preferably around, equal to, or below 0.1. Possible drying devices comprise convection ovens, belt dryers, vacuum dryers, fluidized bed dryers, rotary dryers, just to mention a few.

The moment of drying in the process of obtaining the pellets is not crucial. For example, the drying may take place after compaction, if compaction is easier with slightly moistened components. However, the above-indicated Aw values are preferably achieved before the coating, to the end that. the low Aw values are preserved within the coated pellets.

The number of viable cells (cfu) added to the premix of the inner matrix is dependent on the intended consumption of pellets per day, which in turn is also dependent on the size of the pellet and/or the number of pellets added to a food product. Further variables are the density or occurrence the pellets will finally have in the food product, the concentration of the micro-organisms in a dried form, the serving size of the food product, just to mention a few.

The skilled person is instructed to calculate the number of cfu to be added to the mixture by being aware that the daily dose of probiotics is consumed.

If one pellet is intended to comprise all probiotics of an entire daily serving, or if no other meal comprising probiotics is intended to be consumed than one pellet, the pellet preferably comprises the daily dose of probiotics. In this case, one pellet comprises about 10⁵ to 10¹⁴, preferably 10⁶ to 10¹³, more preferably 10⁷ to 10¹¹ cfu/day.

If, alternatively, the expected daily consumption of pellets comprises 2 to 10 pellets/day, the above-indicated ranges of cfu per pellet may be divided by the corresponding number.

Advantageously, the amount of added micro-organism is calculated in a way that an effective amount of micro-organism will be consumed by consuming one or two, optionally three servings of the respective food product to which the pellets will be added.

If a binder, lubricant and/or plasticizer is used, it may be selected from the lists above. For example, glycerol, in the range of 0.15 to 20%, preferably 0.5 to 10%, calculated as the total weight of the inner matrix, may be sprayed onto the surface of inner matrix components and the probiotic preparation.

Upon the optional addition of a binder, lubricant and/or plasticizer, which may already be added to the premix before the drying step above, the resulting mix may be compacted, for example at a sufficient compaction pressure. Usually, it may be conceived that very high pressures, for example pressures that are substantially above 10'000bar should be avoided, because the bacteria may be destroyed. However, the (probiotic) bacteria, depending on their condition (dried, wet, on a carrier) may support varying pressures.

The lower limit of a compaction pressure is dependent on the "compaction"-properties of the inner matrix. In principle, the compaction pressure may be adjusted following the criterion that a reasonable consistency and/ or stability of the pellet is obtained by compaction.

The upper limit is principally unidentified, but if the food product is intended for being chewed, the compaction pressure is preferably selected in a way that the compacted pellets will not be too hard to avoid damages of teeth.

Besides the fact that the compaction pressure may be selected from a broad range, it was found that pressures in the range of 100 to 10'000bar, preferably 200 to 9000bar and more preferably 300 to 8000 bar. For example, compaction may be performed at a pressure in the range of 600 to 8000 bar. The pressures indicated above strongly depend on the status of the micro-organism. If they are still moist, pressures above 4500 bar may destroy them. However, if the probiotics are in the form of spores, much higher pressures may be applied.

Compaction may be achieved by any suitable compacting device. Examples are rotary tablet presses, eccentric tablet presses, single and double punch tablet presses, single and multi-layer tablet presses, briquetting mills, pellet mills, for example.

The pellets may have the volumes as indicated above and be of any suitable, adequate or desired form. For example, they may have the form of spheres, cubes, pyramids, tablets or any classic, modified or complex three-dimensional form. Furthermore they may have a form that corresponds to the food product to which the pellets are added. For example, if the pellets are added to a pet food for dogs, they may have the form of bones, animals, cats or other forms that fit well with the food product.

Depending on the components that accompany the micro-organisms, the general process for compacting the mixture may be freely modified, supplemented and adjusted.

If the Aw of the compacted matrix is not yet sufficiently low (see values given above) a drying step should be introduced before the following coating step (see above).

Then, the compacted pellets comprising the inner matrix may be coated to further protect the micro-organism from deleterious effect of subsequent absorption of water during the shelf-life of the food product. The coating may be done by any suitable coating technique, for example, spraying, melt or solvent coating equipment, fuidized bed coater, drum coater or pan coater, just to mention a few. The pellets are coated with moisture barrier, which is preferably foodgrade, as already exemplified.

Preferably, the amount of coating may provide from 2 to 30%, preferably 5 to 20%, more preferably 8 to 18% of the uncoated pellets.

It is understood for the purpose of the present invention that the mentioned coating process can be carried out either in one or in multiple steps and that the term "moisture barrier" refers to either a single layer of one compound or a mixture of compounds or to multiple layers of one or more compounds with said barrier properties.

Due to the coating, the drying of most or all of the components of the inner matrix, and the compaction, a low Aw of the pellets may be maintained for a prolonged time.

Thereafter, the food product the probiotics are intended for may be supplemented with a sufficient amount of the pellets according to the invention. The food product may have a moisture content significantly above the Aw of the pellets. For example, if the pellets are added a food product with an Aw of ≥ 0.2, ≥ 0.4, ≥ 0.5 or even ≥ 0.6, a surprisingly high viability over a long storage time is achieved.

The food product may be any food product to which the beneficial function of probiotics is wished to be added. For example, it may be a pet food, including treats. However, it may be any food, intended for any animal. For example, the food product may be a particulate food or food ingredient, such as certain semi-dry pet foods, breakfast cereals, breakfast flakes, -crisps, or -puffs, snacks, chips, dips, biscuits, candies, confectionary, chocolate, bars, muesli, instant beverages in tablet or pellet form, bouillon cubes, instant soup and sauces in tablet or pellet form, oral cosmetics. Of course, the food product may be a food product that is manufactured with particulate ingredients, such as certain bars, which consist of compressed particulate ingredients, for example.

As hinted at above, the number/weight of pellets intended to be added to a food product depends on several factors, amongst which (I) the cfu/g of pellets, (II) the serving size of the food product, and (III) the "effective dose", that is, the amount of cfu that preferably be consumed to obtain the desired effect. The effective daily dose of many probiotics with respect to many animals lies in the range of 10⁷ to 10¹⁰, for example between 10⁸ to 10⁹ cfu per day and individual.

For example, the pellets may used exclusively, that is, the pellets form the entire food product, for example as a treat or a supplement. In the instance of a particulate pet food, this would mean that all particles of the food are constituted by the pellet according to the invention.

In another example, the pellet may be added to a food product in an amount of 1 to 70%, preferably 3 to 50%, more preferably 5 to 30% and most preferably 8 to 20% to a food product. These percentages may be by weight or by number of particles, such as pet food kibbles, for example.

The following examples are given by way of illustration only and in no way should be construed as limiting the subject matter of the present application. It is repeated that the gist of the present invention resides also in the fact that an unlimited variety of food ingredients may be used to form the inner matrix of the pellets. Percentages and parts are by weight unless otherwise indicated.

### Example 1: Preparation of Pellets for Pet Food.

Pellets are prepared by compaction of a powder matrix and are coated with a food grade component providing a high moisture barrier. The entire mixture comprises chicory flour; maltodextrin (DE2-6), and FRISKIES Vitality®, a semi-humid pet food for dogs that is commercially available, foodgrade binders, and a dried bacterial preparation of a *Enterococcus faecium* strain.

First, a premix was prepared of chicory flour (50% of premix), and powdered FRISKIES Vitality® (25% of premix). This premix was dried in a convetion oven to a Aw close to zero (Aw< 0.01), and moist maltodextrin (Aw about 0.3, 25% of premix) was mixed in to complete the premix.

Glycerol (3% of the weight of the premix) was sprayed on the surface of the premix powder to plasticize the surface of the powder particles.

By addition of the bacterial preparation, the mixture was completed.

The mixture was compacted to a cylindrical pellet (diameter: 1cm, height about 1cm) with a slightly convex top and bottom, with a single-punch hydraulic laboratory press (Beckmann PT16). Compaction pressure was 7.6 ton/cm². The water activity of the pellet was 0.084 at 25°C.

Half of the pellets were coated with a fat-based moisture barrier (Witocan 42/44 pastillen, Condea, France. Four coating layers were applied by dipping the pellets in a melt of the barrier material (temperature about 50°C). The total amount of coating was about 15% of the uncoated pellets.

A schematic view of the coated pellets is given in Figure 1.

### Example 2 : Recovery of Micro-organisms after Exposure to Humidity

The stability of coated pellets according to Example 1 were compared with the stability of micro-encapsulated *E. faecium* NCIMB 10415 (commercialised as LBC-ME10) obtained from Cerbios-Pharma, Lugano, Switzerland, , comprising about 5 x 10E+10 cfu/g . The micro-capsules comprise the probiotic strain on a sucrose core that is then coated with several layers of undefined substances (food-grade moisture barriers) and the process to obtain these micro-capsules is largely unknown. The micro-capsules are known to persist for a long time in semi-humid environment and are considered to be the best product currently available on the market.

Hence, the coated pellets according to Example 1 and micro-encapsulated *E. faecium* NCIMB 10415 were exposed for 60 days to 30°C and a humid environment (relative humidity of 70%). After different intervals, samples were taken and viable cell counts of *E. faecium* NCIMB 10415 contained in the pellets and in the micro-capsules were determined

In Figure 2 the recovery rate (in % of the initial cell count) of *E. faecium* SF68 in both samples is shown.

Remarkably, the compacted pellets performed better than the commercially available micro-capsules, especially after a storage time of 20 days. The recovery rate in the commercially available product decreased strongly and constantly, whereas the decrease in recovery of micro-organisms is prominently slower in the pellets according to the invention.

## Claims

1. A pellets comprising a compacted inner matrix and at least one coating, wherein the inner matrix comprises viable micro-organisms and the coating comprises a moisture barrier, **characterised in that** the pellet has a volume of at least 0.02cm³.

2. Pellet according to claim 1, wherein the inner matrix, before or shortly after the coating, is **characterised by** a water activity of below 0.3.

3. Pellet according to claim 1, wherein the inner matrix has an envelope density of more than 0.8g/cm³.

4. Pellet according to claim 1, which comprises 10⁵ to 10¹⁴ viable micro-organisms.

5. Pellet according to claim 1, wherein the inner matrix further comprises at least one component selected from the group of fillers, functional ingredients, lubricants, plasticizers, food-grade binders, and combinations thereof.

6. Pellet according to claim 1, wherein the inner matrix further comprises ingredients selected from the group of digestible starches, resistant starches, other fibre, milled cereals, dried and milled vegetables, cellulose and cellulose derivatives, pet food, maltodextrin, chicory flour, protein isolates, yeast extracts, and mixtures thereof.

7. Pellet according to claim 1, wherein the coating comprises a food-grade moisture barrier.

8. Pellets within a food product, wherein the pellets are the pellets according to any of claim 1 to 7 and the inner matrix of the pellets comprises at least one component of the food product.

9. Delivery system of probiotics, which comprises the pellets according to any of claim 1 to 8.

10. Use of the pellets of any of claim 1 to 8 as a delivery system for probiotics in a moist, semi-moist, or semi-dry food product.

11. A process for obtaining a pellets to supplement a food product with viable micro-organisms, which comprises the steps of mixing a preparation of micro-organisms and further components, drying the mixture to an Aw below 0.3, compacting the mixture under pressure to obtain pellets comprising a volume of at least 0.02cm³, and coating the pellets with a moisture barrier.

12. The process according to claim 12, wherein the further components comprise at least part of the ingredients of the food product.

13. A food product comprising the pellets according to any of claims 1 to 7, wherein the food product and the inner matrix of the pellet share at least one component, ingredient or constituent.

14. Particulate food, food additive, supplement or pharmaceutical product consisting of a mixture of at least two types of particles, one being the probiotic pellet according to any of claims 1 to 8.
